(19) **Europäisches Patentamt** / **European Patent Office** / **Office européen des brevets**

(11) **EP 4 039 248 B1**

(12) # EUROPEAN PATENT SPECIFICATION

(45) Date of publication and mention of the grant of the patent:
**28.01.2026   Bulletin 2026/05**

(21) Application number: **21461508.0**

(22) Date of filing: **04.02.2021**

(51) International Patent Classification (IPC):
*A61K 9/08* (2006.01)        *A61K 47/26* (2006.01)
*A61K 31/728* (2006.01)

(52) Cooperative Patent Classification (CPC):
(C-Sets available)
**A61K 9/0048; A61K 9/08; A61K 31/4172;**
**A61K 31/728; A61K 47/26**        (Cont.)

(54) **OPHTHALMIC COMPOSITION**

OPHTHALMISCHE ZUSAMMENSETZUNG

COMPOSITION OPHTALMIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**10.08.2022   Bulletin 2022/32**

(73) Proprietor: **Zaklady Farmaceutyczne Polpharma S.A.**
**83-200 Starogard Gdanski (PL)**

(72) Inventors:
• **Kubisiak, Marcin**
  **02-315 Warszawa (PL)**
• **Ratajczak, Tomasz**
  **02-315 Warszawa (PL)**
• **Popielska, Iwona**
  **02-315 Warszawa (PL)**
• **Rochowczyk, Anna**
  **02-315 Warszawa (PL)**
• **Magnuszewska, Monika**
  **02-315 Warszawa (PL)**
• **Domel, Izabela**
  **02-315 Warszawa (PL)**

(74) Representative: **Obrycki, Pawel Andrzej**
**Zaklady Farmaceutyczne Polpharma SA**
**Ul. Pelplinska 19**
**83-200 Starogard Gdanski (PL)**

(56) References cited:
EP-A1- 1 860 116        EP-A1- 3 072 503
EP-B1- 3 072 503        WO-A1-03/049747
WO-A1-2004/064866        WO-A1-2010/056113
WO-A1-2015/136186        CN-A- 107 440 975
CN-A- 107 913 246

• SILVANI LUDOVICA ET AL: "Arabinogalactan and hyaluronic acid in ophthalmic solution: Experimental effect on xanthine oxidoreductase complex as key player in ocular inflammation (in vitro study)", EXPERIMENTAL EYE RESEARCH, ACADEMIC PRESS LTD, LONDON, vol. 196, 4 May 2020 (2020-05-04), XP086177802, ISSN: 0014-4835, [retrieved on 20200504], DOI: 10.1016/J.EXER.2020.108058

Remarks:
The file contains technical information submitted after the application was filed and not included in this specification

EP 4 039 248 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

(52) Cooperative Patent Classification (CPC): (Cont.)

C-Sets
**A61K 31/4172, A61K 2300/00;**
**A61K 31/728, A61K 2300/00**

**Description**

**Technical Field**

The present invention relates to the field of compositions for the prevention and/or treatment of ophthalmic pathologies, in particular for dry eye syndrome.

**Background Art**

Dry eye syndrome, also known as dry eye or dry keratoconjunctivitis (KCS), is a condition in which the amount of tears produced by the lacrimal glands is insufficient or tear film evaporates excessively. The reduced flow of tears can result in the formation of an inadequate or no tear film on the surface of the eye. The tear film acts inter alia as a slip or lubricating agent between the eyelid and the surface of the eye. As a result of the absence of or inadequate tear film, the epithelial layers can suffer from considerable trauma. This results in discomfort in the eye which is accompanied by itching, tingling and / or burning sensations.

Eye dryness can result from an abnormality of the lacrimal glands, inflammation of the eyelids, ocular inflammation due to an allergy, refractive surgery (including laser), a deficiency of certain lipids in the eye, daily diet, prolonged contact lens wearing, hormonal alteration, autoimmune disease, or secondary effects of certain drugs.

In the treatment of dry eye, the application of saline or artificial tears, also called lubricating or moistening eye drops, is sometimes recommended. Artificial tears of low to medium viscosity are usually based on polyvinyl alcohols or cellulosic derivatives. These remedies provide no more than some local relief for a short time and, besides, they have the disadvantage of changing the cornea surface decreasing its density (Wegener, A. R., Meyer, L. M. & Schönfeld, C. L. Effect of viscous agents on corneal density in dry eye disease. J. Ocul. Pharmacol. Ther. 31, 504-508 (2015).

Eye drops containing hyaluronic acid, natural biopolymer possessing excellent viscoelasticity, high moisture retention capacity and hygroscopic properties, are widely used in the context of the treatment of dry eye. Hyaluronic acid is used in eye drops for the hydration of the cornea. It forms a transparent, lubricating and wetting film on the surface of the eye, protecting the cornea against drying. These properties allow effective relief of clinical signs. However, prior art eye drops containing hyaluronic acid have the problem of limited action time, forcing the patient to repeat the instillations frequently. Moreover, it is known that administration of hyaluronic acid in the eye produces a slight discomfort which lasts from a few seconds to a few minutes after instillation and that is often accompanied by a slight blur in the vision.

Dry eye syndrome is a common disease associated to eyes inflammation, irritation and tear film instability. An artificial drop formulation based on arabinogalactan and hyaluronic acid has been proposed in the prior art. This prior art formulation also comprises panthenol and trehalose (L. Silvani, A. Bedei, G. De Garcia, S. Remiddi; Arabinogalactan and hyaluronic acid in ophthalmic solution: Experimental effect on xanthine oxidoreductase complex as key player in ocular inflammation (in vitro study); Experimental Eye Research 196 (2020) 108058).

In conclusion, hyaluronic acid eye drops are more effective for mild to moderate eye dryness than saline or artificial tears based on polyvinyl alcohols or cellulosic derivatives. Patients with severe drought may also find some relief but are generally forced to turn to different products containing further active ingredients such as immunosuppressants (e.g. ciclosporin) or anti-inflammatories (e.g. lifitegrast). Use of these further active ingredients, however, may have undesired side effects, particularly when used during prolonged time.

The prior art has provided compositions which combine hyaluronic acid with further ingredients in order to improve the treatment of dry eye. However, so far difficulties have been encountered in developing a convenient solution that overcomes the drawbacks mentioned above.

Thus, there exists the need to provide improved compositions for the treatment of dry eye syndrome and related pathologies.

**Summary of Invention**

The inventors have developed an ophthalmic composition for the treatment of ophthalmic pathologies, in particular dry eye syndrome, that overcomes the drawbacks mentioned above. Said composition comprises a combination of hyaluronate with panthenol, oligosaccharide, and carnosine or its derivative, and combinations thereof, which achieves multiple advantages.

A first aspect of the invention thus refers to an ophthalmic composition comprising:

- hyaluronic acid or an ophthalmologically acceptable salt thereof in amount of less than or equal to 2% by weight and not less than or equal to 0.01% by weight,
- an oligosaccharide in amount of less than or equal to 6% by weight and not less than or equal to 0.1% by weight,
- a compound selected from the group consisting of panthenol, pantothenic acid, an ophthalmologically acceptable salt of pantothenic acid, and combinations thereof in amount of less than or equal to 5% and not less than or equal to 0.1% by weight, and an amount of water up to 100% by weight of the composition.

The composition of the invention comprises also carnosine or its derivative, and combinations thereof.

Carnosine and derivatives thereof such as carcinine, anserine, balenine, N-acetylcarnosine are known to be among the most important and potent natural human and water-soluble antioxidant agents which act as universal antioxidants both in the lipid phase of cellular and biological membranes and in the aqueous environment protecting lipids and water-soluble molecules especially proteins (including enzymes). Carnosine is also able to avoid carbonylation and glycosylation. This combination of effects of avoiding oxidation, carbonylation and glycosylation is a unique combination. Within the current invention, the term "carnosine" also includes such compounds like carcinine, anserine, balenine and N-acetylcarnosine. However, preferably N-acetylcarnosine is used.

Finally, the present composition is highly effective for treating dry eye and related conditions preferably without the need of additional active ingredients or additional excipients or carriers, and requires no preservatives. The absence of preservatives and/or additional active agents, excipients or carriers guarantees lack of undesired side effects, such as allergic reactions, secondary dry eye, trabecular meshwork degeneration, mitochondrial dysfunction and ocular inflammation.

Altogether, the ophthalmic composition of the invention outperforms prior art compositions by adequately balancing moisturizing with remanence, lack of discomfort and lack of undesired side effects. An ophthalmic composition is therefore provided which is effective for the prevention and/or treatment of an ophthalmological pathology, such as dry eye syndrome, Sjögren syndrome, inflammation of the conjunctiva, irritation, itchiness, burning and allergic reactions of the eye, and related conditions, while being highly convenient for the user. The expression "related conditions" in the present application is understood as conditions which are related to a deficient lubrication of the surface of the eye.

A second aspect of the invention thus refers to an ophthalmic composition as defined in the first aspect of the invention for use in prevention and/or treatment of an ophthalmological pathology selected from the group consisting of dry eye syndrome, Sjögren syndrome, inflammation of the conjunctiva, irritation, itchiness, burning and allergic reactions of the eye, and related conditions. This may also be formulated as an ophthalmic composition as defined in the first aspect of the invention for the preparation of a medicament for prevention and/or treatment of an ophthalmological pathology selected from the group consisting of dry eye syndrome, Sjögren syndrome, inflammation of the conjunctiva, irritation, itchiness, burning and allergic reactions of the eye, and related conditions. The invention also refers to a method for prevention and/or treatment of dry eye syndrome, Sjögren syndrome, inflammation of the conjunctiva, irritation, itchiness, burning and allergic reactions of the eye, and related conditions comprising administering a therapeutically effective amount of an ophthalmic composition as defined in the first aspect of the invention, in a subject in need thereof, including a human.

## Detailed description of the invention

The present invention provides an aqueous ophthalmic composition comprising a synergic combination of hyaluronic acid or an ophthalmologically acceptable salt thereof, an oligosaccharide, and a compound selected from the group consisting of panthenol, pantothenic acid, an ophthalmologically acceptable salt of pantothenic acid, carnosine or its derivative, and combinations thereof.

Hyaluronic acid (CAS Registry Number: 9004-61-9) is a natural substance which can be found in the eye but also in other parts of the body. It is a long-chain polymer of disaccharide units of glucuronic acid and N-acetylglucosamine linked via alternating $\beta$-(1$\rightarrow$4) and $\beta$-(1$\rightarrow$3) glycosidic bonds. Hyaluronic acid can be 25,000 disaccharide repeats in length. Polymers of hyaluronic acid can range in size from 5,000 to 20,000,000 Da in vivo. In the eye it ensures that a uniform, stable and particularly long-term-adhering moisture film is formed on the surface which cannot be rinsed off rapidly.

Several ophthalmologically acceptable salts of hyaluronic acid are known which can be used in the composition of the invention, the most common of which is sodium hyaluronate (herein also termed as HA, PubChem CID: 23663392). The term "ophthalmologically acceptable salts" encompasses any salt formed from ophthalmologically acceptable nontoxic acids including inorganic or organic acids. There is no limitation regarding the salts, except that if used for therapeutic purposes, they must be ophthalmologically acceptable.

The concentration of hyaluronic acid or an ophthalmologically acceptable salt thereof in the composition of the invention is less than or equal to 2 % by weight. In some aspects of the invention the concentration of hyaluronic acid or an ophthalmologically acceptable salt thereof in the composition of the invention is less than or equal to 0.6% by weight. In further aspects the concentration of hyaluronic acid or an ophthalmologically acceptable salt thereof in the composition of the invention is less than or equal to 0.5% by weight. The concentration of hyaluronic acid or an ophthalmologically acceptable salt thereof in the composition of the invention is not less than or equal to 0.01% by weight. In some aspects, the concentration of hyaluronic acid or an ophthalmologically acceptable salt thereof in the composition of the invention is not less than or equal to 0.1% by weight. In further aspects, the concentration of hyaluronic acid or an ophthalmologically acceptable salt thereof in the composition of the invention is not less than or equal to 0.15% by weight. In other aspects, the composition comprises 0.3 to 0.4 % by weight of hyaluronic acid or an ophthalmologically acceptable salt thereof. In some aspects the composition comprises 0.4 % by weight of hyaluronic acid or an ophthalmologically acceptable salt thereof.

In the present application, "% by weight" means % w/v, i.e. percentage by weight of the ingredient with respect to the total volume of the composition. In other words, x to y % by weight of ingredient A represents x to y grams of ingredient A in 100 ml of the composition.

Hyaluronic acid and the ophthalmologically acceptable salt thereof may be in the form of cross-linked polymer. Preferably, the polymer is not cross-linked. In addition, the weight average molecular weight (Mw) of the hyaluronic acid or of the ophthalmologically acceptable salt thereof is preferably from 5 to 2500 kDa. In some aspects, the weight average molecular weight (Mw) of the hyaluronic acid or ophthalmologically acceptable salt thereof is from 30 to 1500 kDa. In other aspects, the weight average molecular weight of the hyaluronic acid or ophthalmologically acceptable salt thereof is from 40 to 800 kDa. In some other aspects, the weight average molecular weight of the hyaluronic acid or ophthalmologically acceptable salt thereof is from 50 to 500 kDa. In some further aspects, the weight average molecular weight of the hyaluronic acid or ophthalmologically acceptable salt thereof is from 50 to 90kDa. In some aspects, the weight average molecular weight of the hyaluronic acid or ophthalmologically acceptable salt thereof is about 70kDa. The weight average molecular weight of the hyaluronic acid or of the ophthalmologically acceptable salt thereof may be determined by Size extrusion chromatography - multiangle laser scattering (SEC MALS).

In a particular aspect of the invention, the ophthalmologically acceptable salt of hyaluronic acid is selected from the group consisting of sodium hyaluronate, potassium hyaluronate and combinations thereof. In a further aspect, the ophthalmologically acceptable salt of hyaluronic acid is sodium hyaluronate.

Oligosaccharides are oligomers formed from an n number of monosaccharide units, with n generally between 2 and 10. Oligosaccharide can be linear, branched or cyclic. Oligosaccharides may have intrinsic properties of interest in the ophthalmic field, in particular anti-inflammatory, anti-apoptotic, or ability to regulate osmolarity and therefore water uptake activity. The concentration of oligosaccharide in the composition of the invention is less than or equal to 6% by weight. In some aspects, the composition comprises less than or equal to 5% by weight of oligosaccharide. In other aspects, the composition comprises less than or equal to 4% by weight of oligosaccharide. The concentration of oligosaccharide in the composition of the invention is not less than or equal to 0.1% by weight. In some aspects, the composition comprises not less than or equal to 0.5% by weight of oligosaccharide. In other aspects, the composition comprises not less than or equal to 1% by weight of oligosaccharide. In further aspects, the composition comprises 2 to 3 % by weight of oligosaccharide. In some aspects, the composition comprises 3% by weight of oligosaccharide.

According to some aspects of the invention, the oligosaccharide is a disaccharide which may consist of two identical (homo) or different (hetero) monomers. A non-limiting example is a disaccharide selected from the group consisting of cellobiose, gentiobiose, inulobiose, isomaltose, isomaltulose, kojibiose, lactulose, lactose, laminaribiose, leucrose, maltose, maltulose, 2 alpha-mannobiose, 3 alpha-mannobiose, melibiose, nigerose, rutinose, sophorose, sucrose, trehalose, trehalulose, turanose, and any combination thereof. In a particular aspect, the oligosaccharide is a disaccharide which is trehalose. In further aspects, trehalose is used as trehalose anhydrous and/or trehalose dihydrate. In the most particular aspect, trehalose is used as trehalose dihydrate.

The composition according to the invention comprises less than or equal to 5% by weight of a compound selected from the group consisting of panthenol, pantothenic acid, an ophthalmologically acceptable salt of pantothenic acid, and combinations thereof. In some aspects, the composition comprises less than or equal to 4% by weight, or less than or equal to 3 % by weight, for example 2% by weight, of a compound selected from the group consisting of panthenol, pantothenic acid, an ophthalmologically acceptable salt of pantothenic acid, and combinations thereof. The composition according to the invention comprises not less than or equal to 0.1% by weight of a compound selected from the group consisting of panthenol, pantothenic acid, an ophthalmologically acceptable salt of pantothenic acid, and combinations thereof. In some aspects, the composition comprises not less than or equal to 0.5% by weight, or not less than or equal to 1%, of a compound selected from the group consisting of panthenol, pantothenic acid, an ophthalmologically acceptable salt of pantothenic acid, and combinations thereof.

Panthenol (also called pantothenol) (CAS number: 16485-10-2, PubChem CID: 4678) is the alcohol analog of pantothenic acid (vitamin B5, CAS number: 599-54-2), and is thus a provitamin of B5. In organisms it is quickly oxidized to pantothenic acid. In particular aspects of the invention, the compound is D-panthenol (herein also termed as dexpanthenol or DEX, CAS number: 81-13-0; PubChem CID: 131204). In other particular aspects, the compound is an ophthalmologically acceptable salt of pantothenic acid which can be selected from sodium pantothenate and calcium pantothenate.

The ophthalmic composition of the invention may have a weight ratio of hyaluronic acid or an ophthalmologically acceptable salt thereof, to oligosaccharide, to the compound selected from panthenol, pantothenic acid, an ophthalmologically acceptable salt of pantothenic acid, and combinations thereof that is from 1:1:1 to 1:50:40. In particular aspects, the weight ratio is from 1:2.5:2.5 to 1:27:20. In other aspects, the weight ratio is from 1:3:3 to 1:22:18. In other further aspects, the weight ratio is from 1:3.5:3.5 to 1:15:12, for example 1:7.5:5. Furthermore, in one of the aspects the ophthalmic composition of the invention comprises an oligosaccharide in an amount by weight not less than or equal to an amount by weight of a compound selected from panthenol, pantothenic acid, an ophthalmologically acceptable salt of pantothenic acid, and combinations thereof. In a further aspect, the ophthalmic composition of the invention comprises an oligosaccharide in an amount by weight not less than an amount by weight of a compound selected from panthenol, pantothenic acid, an ophthalmologically acceptable salt of pantothenic acid, and combinations thereof.

The composition according to the invention comprises carnosine or its derivative, and combinations thereof. Carnosine and derivatives thereof such as carcinine, anserine, balenine, N-acetylcarnosine are known to be among the most important and potent natural human and water-soluble antioxidant agents which act as universal antioxidants both in the lipid phase of cellular and biological membranes and in the aqueous environment protecting lipids and water-soluble molecules especially proteins (including enzymes). Carnosine is also able to avoid carbonylation and glycosylation. This combination of effects of avoiding oxidation, carbonylation and glycosylation is a unique combination. Within the current invention, the term "carnosine" also includes such compounds like carcinine, anserine, balenine an N-acetylcarnosine. However, preferably N-acetylcarnosine is used.

The composition according to the invention may comprise less than or equal to 5% by weight of carnosine or its derivative, and combinations thereof. In some aspects, the composition comprises less than or equal to 3% by weight, or less than or equal to 2% by weight, for example 1% by weight of carnosine or its derivative, and combinations thereof. The composition according to the invention may also comprise not less than or equal to 0.1% by weight of carnosine or its derivative, and combinations thereof. In some aspects, the composition comprises not less than or equal to 0.2% by weight, or less than or equal to 0.5% by weight, of carnosine or its derivative, and combinations thereof.

The composition according to the invention may further also contain an additive selected from the group of nonionic type isotonizing agents, antioxidants and / or buffer systems. According to another particular aspect, the composition of the invention is an isotonic or hypotonic formulation with an osmolality advantageously between 170 and 350 mOsmol/kg. According to a further particular aspect, the composition of the invention has an osmolarity not more than 350 mOsmol/kg, preferably not more than 330 mOsmol/kg, most preferably not more than 320 mOsmol/kg. Regarding the lower end of range the osmolarity is preferably not less than 170 mOsmol/kg, more preferably not less than 220 mOsmol/kg, most preferably not less than 260 mOsmol/kg. The osmolarity of the composition of the invention can be 270 mOsmol/kg, 285 mOsmol/kg or 290 mOsmol/kg. The indicated values of osmolarity correspond to the values measured using Osmo1® Single-Sample Micro-Osmometer which use the gold-standard freezing point depression method technology. This characteristic can be obtained, for example, by addition of osmolarity regulating agent such as sodium chloride (NaCl), potassium chloride (KCl) and/or calcium chloride ($CaCl_2$). Thus, in particular aspects the composition of the invention further comprises sodium chloride and/or potassium chloride and/or calcium chloride. The sodium chloride may be present in the composition from 0.01 to 0.1 % by weight. In some aspects, the composition comprises from 0.03 to 0.06 %, or from 0.04 to 0.05 % by weight of sodium chloride. In some further aspects, the composition of the invention may comprise sodium chloride from 0.03 to 0.06 % by weight, potassium chloride from 0.003 to 0.02 % by weight and calcium chloride from 0.0002 to 0.0108 % by weight.

Furthermore, the ophthalmological composition may further benefit from comprising at least one buffer system, for example a buffer selected from the group consisting of borate buffer, citrate buffer, phosphate buffer, tris buffer, trometamol/maleic acid. In particular aspects, the composition comprises citrate buffer. However, in some aspects the composition is devoid of a buffer system.

The pH of the composition of the invention may be from 5 to 9. In particular aspects, the pH is from 5.5 to 7.5, or from 6 to 7. For example, the pH of the composition of the invention may be from 6.2 to 6.3. The pH of the composition may be determined by a pH-meter or a pH strip. In particular the pH ranges given above may be determined by a pH-meter WTW pH 1970i. The preferred pH value may be obtained by addition of pH regulating agent such as hydrochloric acid (HCl) and/or sodium hydroxide (NaOH).

According to a particularly preferred aspect, the composition according to the invention is devoid of preservatives. By preservative it is understood any substance which can be used as ophthalmological preservative, such as e.g. the preservatives listed further on. Preservatives can damage the precorneal tear film and lead to a reduction in the number of microvilli and microplicae of the surface corneal epithelium cells, which results in irritation and/or inflammation of the eye. By dispensing with preservatives in the solution according to the invention, such irritation and/or inflammation can be avoided or at least reduced. In particular the composition of the invention is devoid of benzalkonium chloride, which is the most frequently used preservative in eye drops. The composition of the invention is also devoid of phosphates. According to the literature phosphates increase the risk of corneal tissue calcification leading to burning eye and cornea opacity.

It is also preferred that the ophthalmic composition according to the invention contains no further pharmaceutically active ingredients. In particular, the composition of the invention is devoid of anti-inflammatory agents and/or immune-suppressants. In particular aspects, the composition is devoid of further excipients and carriers. Particular aspects thus refer to a composition comprising hyaluronic acid or an ophthalmologically acceptable salt thereof, an oligosaccharide, and a compound selected from the group consisting of panthenol, pantothenic acid, an ophthalmologically acceptable salt of pantothenic acid, and combinations thereof as the only active ingredients. A composition according to the invention also comprises carnosine and/or its derivative as a further active ingredient. In some aspects, the ophthalmic composition according to the invention may further comprise an osmolarity regulating agent such as NaCl, KCl and/or $CaCl_2$, a pH regulating agent such as HCl and/or NaOH and water. These compositions could also contain a buffer, for example citric buffer. So in preferred aspects the composition of current invention is devoid of preservatives, further active agents and further excipients and carriers.

In one of the aspects, the ophthalmic composition comprises sodium hyaluronate, trehalose, D-panthenol and N-acetylcarnosine as the only active ingredients, water and, optionally, an osmolarity regulating agent such as NaCl and/or pH regulating agent such as HCl and/or NaOH and/or buffering agent, for example citric buffer. Said compositions are preferably devoid of preservatives, phosphates, further active agents and further excipients and carriers. In a further aspect, the composition of the invention consists essentially of sodium hyaluronate, trehalose, D-panthenol and N-acetylcarnosine as the only active ingredients, water and, optionally, an osmolarity regulating agent such as NaCl and/or pH regulating agent such as HCl and/or NaOH. The weight average molecular weight (Mw) of sodium hyaluronate is preferably from 5 to 2500 kDa. In some aspects, the weight average molecular weight (Mw) of sodium hyaluronate is from 30 to 1500 kDa. In other aspects, the weight average molecular weight (Mw) of sodium hyaluronate is from 40 to 800 kDa. In some other aspects, the weight average molecular weight (Mw) of sodium hyaluronate is from 50 to 500 kDa. In some further aspects, the weight average molecular weight of sodium hyaluronate is from 50 to 90kDa. In one of the aspects, the weight average molecular weight of sodium hyaluronate is about 70kDa.

Advantageously, the composition according to the invention has a dynamic viscosity suitable for the targeted topical ophthalmic application. It has been determined that easy and non-threatening removal through lacrimal duct is better when dynamic viscosity of the ophthalmic solution / tear mixture is less than 50 mPa•s and this is accomplished by the composition of the current invention. Thus, in some aspects, the composition according to the invention has dynamic viscosity at 20 ° C from 1 to 50 mPa•s. In particular aspects the viscosity is from 1 to 20 mPa•s, preferably from 1.5 to 10 mPa•s. These values correspond to the values measured using a BROOKFIELD DV3T rotating viscometer at 20°C.

The synergic effects achieved by the composition according to the invention also have a positive effect on the production costs of the composition. The referred ingredients are expensive raw materials. The synergistic effect of water-binding capacity proves to be very advantageous for saving on raw materials and costs. As a result of the synergistic effect, a selected water-binding capacity can be produced with a lower amount of raw material than in the case of the ophthalmological compositions from the state of the art, as a result of which cost savings can be made. Additionally, lower amount of active ingredients has the advantage of producing less undesired side effects.

As mentioned above, it may be convenient that the composition of the invention is devoid of further active ingredients. However, in other aspects, the composition according to the invention may also contain one or more active agents in therapeutically effective amounts. Said active agents may be for example selected from antibiotics, antibacterials, antifungals, antivirals, immunosuppressants, anti-inflammatories or anti-laucomatous agents such as prostaglandins, beta-blockers, inhibitors carbonic anhydrase or alpha-adrenergic agonists. Such an active agent may especially be chosen from the following group: aceclidine, acetazolamide, aciclovir, anecortave, apraclonidine, atropine, azapentacene, azelastine, bacitracin, befunolol, betamethasone, betaxolol, bimatoprost, brimonidine, brinzolamide, carbachol, cardolol, celecoxib, chloramphenicol, chlortetracycline, ciprofloxacin, cromoglycate, cyclopentolate, cyclosporine, dapiprazole, demecarium, dexamethasone, diclofenac, dichlorphenamide, dipivefrin, dorzolamide, echothiophale, emedastine, epinastine, epinephrine, erythromycin, ethoxzolamide, eucatropine, fludrocortisone, fluorometholone, flurbiprofen, fomivirsen, framycetin, ganciclovir , gatifloxacin, gentamycin, homatropine, hydrocortisone, idoxuridine, indomethacin, isoflurophate, ketorolac, ketotifen, latanoprost, levobetaxolol, levobunolol, levocabastine, levofloxacin, lodoxamide, loteprednol, medrysone, methazolamide, metipranolol, moxifloxacin, naphazoline, natamycin, nedocromil, neomycin, norfloxacin , ofloxacin, olopatadine, oxymetazoline, pemirolast, pegaptanib, phenylephrine, physostigmine, pilocarpine, pindolol, pirenoxine, polymyxin B, prednisolone, proparacaine, ranibizumab, rimexolone, scopolamine, sezolamide, squalamine, sulfacetamide, suprofen, tetracaine, tetracyclin, tetrahydrozoline, tetryzoline, timolol , tobramycin, travoprost, triamcinulone, trifluoromethazolarnide, trifluridine, trimethoprim, tropicamide, unoprostone, vidarbine, xylometazoline, pharmaceutically acceptable salts, or combinations thereof. Quaternary ammonium compounds, especially benzalkonium chloride or polyquad, optionally in combination with EDTA, are generally used as preservatives. Other conservatives are for example: preservatives based on guanidine (PHMB, chlorhexidine); benzyl alcohol; parabens (methyl, ethyl, propyl, ...); mercury-based preservatives, such as thimerosal; chlorobutanol; benzethonium chloride; oxidative preservatives (Purite, ...).

The ophthalmic composition according to the invention may be conveniently configured in the form of eye drops. The composition may be configured in the form of a single-use (single-dose) or multi-dose vial made of, for example low-density polyethylene (LDPE), advantageously of EP quality containing no additives. Non-limiting examples of single-use vials (single dose) or multi-dose vial to deliver eye drops without preservatives for several days are Abak®, Comod®, OSD®, Novelia® or System 3K®. Apart from eye drops, other non-limiting dosage forms for the composition of the invention are cream, gel, hydrogel, lotion, ointment and spray, all of which for application to the eyes. When used, excipients and carriers incorporated into the composition of the invention must be ophthalmologically acceptable. "Ophthalmologically acceptable excipients or carriers" refers to ophthalmologically acceptable materials, compositions or vehicles. Each component must be ophthalmologically acceptable in the sense of being compatible with the other ingredients of the ophthalmic composition. It must also be suitable for use in contact with the eye without excessive toxicity, irritation, allergic response, immunogenicity or other problems or complications commensurate with a reasonable benefit/risk ratio.

A second aspect of the invention refers to an ophthalmic composition as above for use in the prevention and/or treatment of an ophthalmological (or ocular) pathology. In particular aspects, the ophthalmological pathology is selected from the group consisting of dry eye syndrome, Sjögren syndrome, inflammation of the conjunctiva, irritation, itchiness, burning and allergic reactions of the eye, and related conditions. In a particular aspect, the ophthalmic pathology is dry eye syndrome.

The second aspect may also be contemplated as hyaluronic acid or an ophthalmologically acceptable salt thereof for use in the prevention and/or treatment of an ophthalmological (or ocular) pathology when used in combination with an oligosaccharide and a compound selected from the group consisting of panthenol, pantothenic acid, an ophthalmologically acceptable salt of pantothenic acid, and carnosine or its derivative, and combinations thereof. This may also be worded as hyaluronic acid or an ophthalmologically acceptable salt thereof for the preparation of a medicament for the prevention and/or treatment of an ophthalmological (or ocular) pathology when used in combination with an oligosaccharide and a compound selected from the group consisting of panthenol, pantothenic acid, an ophthalmologically acceptable salt of pantothenic acid, and carnosine or its derivative, and combinations thereof.

The second aspect may also be contemplated as an oligosaccharide for use in prevention and/or treatment of an ophthalmological (or ocular) pathology when used in combination with hyaluronic acid or an ophthalmologically acceptable salt thereof and a compound selected from the group consisting of panthenol, pantothenic acid, an ophthalmologically acceptable salt of pantothenic acid, and carnosine or its derivative, and combinations thereof. This may also be worded an oligosaccharide for the preparation of a medicament for prevention and/or treatment of an ophthalmological (or ocular) pathology when used in combination with hyaluronic acid or an ophthalmologically acceptable salt thereof and a compound selected from the group consisting of panthenol, pantothenic acid, an ophthalmologically acceptable salt of pantothenic acid, and carnosine or its derivative, and combinations thereof.

The second aspect may also be contemplated as a compound selected from the group consisting of panthenol, pantothenic acid, an ophthalmologically acceptable salt of pantothenic acid, and combinations thereof for use in the prevention and/or treatment of an ophthalmological (or ocular) pathology when used in combination with hyaluronic acid or an ophthalmologically acceptable salt thereof and an oligosaccharide, and carnosine or its derivative. This may also be worded as a compound selected from the group consisting of panthenol, pantothenic acid, an ophthalmologically acceptable salt of pantothenic acid, and combinations thereof for the preparation of a medicament for the prevention and/or treatment of an ophthalmological (or ocular) pathology when used in combination with hyaluronic acid or an ophthalmologically acceptable salt thereof and an oligosaccharide, and carnosine or its derivative,.

The ingredients in the combination may be administered simultaneously, sequentially or separately. Preferably they are administered simultaneously forming part of the same composition.

The composition according to the invention can be used both in humans and in animals. According to a preferred aspect, this composition is intended for topical application, preferably by instillation in the eye.

The dose is adapted to the severity of the pathology but usually consists in administering in the affected body one to several drops of the composition of the invention per day. The composition of the invention makes it possible to space the administrations, in comparison in particular with a composition not containing the triple combination of ingredients. Thus, a dose of less than 5 drops per day and per eye, or even less than 4, 3, 2 or even 1 single drop per day and by that it can be considered.

The present application also contemplates that an ophthalmic composition as defined above provides a basis for formulating sustained release preparations ("drug delivery system"), for improving bioavailability, remanence and hence the effectiveness of the composition, particularly as described above. Thus, another aspect refers to a sustained release ophthalmic formulation comprising the composition of the invention as defined above, as well as its use in the prevention and/or treatment of an ophthalmological pathology, in particular dry eye syndrome.

For reasons of completeness, various aspects of the invention are set out in the following numbered embodiments:

Embodiment 1. An ophthalmic composition comprising:

- hyaluronic acid or an ophthalmologically acceptable salt thereof in amount of less than or equal to 2% by weight and not less than or equal to 0.01% by weight,
- an oligosaccharide in amount of less than or equal to 6% by weight and not less than or equal to 0.1% by weight,
- a compound selected from the group consisting of panthenol, pantothenic acid, an ophthalmologically acceptable salt of pantothenic acid, and combinations thereof in amount of less than or equal to 5% and not less than or equal to 0.1% by weight,

and an amount of water up to 100% by weight of the composition,

- carnosine or its derivative, and combinations thereof.

Embodiment 2. The ophthalmic composition according to embodiment 1, that comprises hyaluronic acid or an ophthalmologically acceptable salt thereof in amount of less than or equal to 0.6% by weight and/or not less than or equal to 0.1% by weight.

Embodiment 3. The ophthalmic composition according to embodiment 2, that comprises hyaluronic acid or an ophthalmologically acceptable salt thereof in amount of less than or equal to 0.5% by weight and/or not less than or equal to 0.15% by weight.

Embodiment 4. The ophthalmic composition according to embodiment 3, that comprises hyaluronic acid or an ophthalmologically acceptable salt thereof in amount of 0.3 to 0.4 % by weight.

Embodiment 5. The ophthalmic composition according to embodiment 4, that comprises hyaluronic acid or an ophthalmologically acceptable salt thereof in amount of 0.4% by weight.

Embodiment 6. The ophthalmic composition according to any of the embodiments 1-5, that comprises an oligosaccharide in amount of less than or equal to 5% by weight and/or not less than or equal to 0.5% by weight.

Embodiment 7. The ophthalmic composition according to embodiment 6, that comprises an oligosaccharide in amount of less than or equal to 4% by weight and/or not less than or equal to 1% by weight.

Embodiment 8. The ophthalmic composition according to embodiment 7, that comprises an oligosaccharide in amount of 2 to 3 % by weight, preferably 3% by weight.

Embodiment 9. The ophthalmic composition according to any of the embodiments 1-8, that comprises a compound selected from the group consisting of panthenol, pantothenic acid, an ophthalmologically acceptable salt of pantothenic acid, and combinations thereof in amount of less than or equal to 4% by weight and/or not less than or equal to 0.5% by weight.

Embodiment 10. The ophthalmic composition according to embodiment 9, that comprises a compound selected from the group consisting of panthenol, pantothenic acid, an ophthalmologically acceptable salt of pantothenic acid, and combinations thereof in amount of less than or equal to 3% by weight and/or not less than or equal to 1% by weight.

Embodiment 11. The ophthalmic composition according to embodiment 9, that comprises a compound selected from the group consisting of panthenol, pantothenic acid, an ophthalmologically acceptable salt of pantothenic acid, and combinations thereof in amount of 2% by weight.

Embodiment 12. The ophthalmic composition according to any of the embodiments 1-11, wherein the weight average molecular weight (Mw) of the hyaluronic acid or ophthalmologically acceptable salt thereof is from 5 to 2500 kDa.

Embodiment 13. The ophthalmic composition according to embodiment 12, wherein the weight average molecular weight (Mw) of the hyaluronic acid or ophthalmologically acceptable salt thereof is from 30 to 1500 kDa.

Embodiment 14. The ophthalmic composition according to embodiment 13, wherein the weight average molecular weight (Mw) of the hyaluronic acid or ophthalmologically acceptable salt thereof is from 40 to 800 kDa.

Embodiment 15. The ophthalmic composition according to embodiment 14, wherein the weight average molecular weight (Mw) of the hyaluronic acid or ophthalmologically acceptable salt thereof is from 50 to 500 kDa.

Embodiment 16. The ophthalmic composition according to embodiment 15, wherein the weight average molecular weight (Mw) of the hyaluronic acid or ophthalmologically acceptable salt thereof is from 50 to 90 kDa, preferably 70 kDa.

Embodiment 17. The ophthalmic composition according to any of the embodiments 1-16, that comprises sodium hyaluronate.

Embodiment 18. The ophthalmic composition according to any of the embodiments 1-17, that comprises oligosaccharide , preferably disaccharide selected from the group consisting of cellobiose, gentiobiose, inulobiose, isomaltose, isomaltulose, kojibiose, lactulose, lactose, laminaribiose, leucrose, maltose, maltulose, 2 alpha-mannobiose, 3 alpha-mannobiose, melibiose, nigerose, rutinose, sophorose, sucrose, trehalose, trehalulose, turanose, and any combination thereof.

Embodiment 19. The ophthalmic composition according to any of the embodiments 1-18, that comprises trehalose.

Embodiment 20. The ophthalmic composition according to any of the embodiments 1-19, that comprises D-panthenol.

Embodiment 21. The ophthalmic composition according to any of the embodiments 1-19, that comprises an ophthalmologically acceptable salt of pantothenic acid selected from sodium pantothenate and calcium pantothenate.

Embodiment 22. The ophthalmic composition according to any of the embodiments 1-21, wherein the weight ratio of hyaluronic acid or an ophthalmologically acceptable salt thereof, to oligosaccharide, to the compound selected from panthenol, pantothenic acid, an ophthalmologically acceptable salt of pantothenic acid, and combinations thereof is from 1:1:1 to 1:50:40.

Embodiment 23. The ophthalmic composition according to the preceding embodiment, wherein the weight ratio is from 1:2.5:2.5 to 1:27:20.

Embodiment 24. The ophthalmic composition according to the preceding embodiment, wherein the weight ratio is 1:3:3 to 1:22:18.

Embodiment 25. The ophthalmic composition according to the preceding embodiment, wherein the weight ratio is 1:3.5:3.5 to 1:15:12.

Embodiment 26. The ophthalmic composition according to the preceding embodiment, wherein the weight ratio is 1:7.5:5.

Embodiment 27. The ophthalmic composition according to any of the preceding embodiments, that comprises an oligosaccharide in an amount by weight not less than or equal to an amount by weight of a compound selected from panthenol, pantothenic acid, an ophthalmologically acceptable salt of pantothenic acid, and combinations thereof.

Embodiment 28. The ophthalmic composition according to any of the preceding embodiments, that comprises an oligosaccharide in an amount by weight not less than an amount by weight of a compound selected from panthenol, pantothenic acid, an ophthalmologically acceptable salt of pantothenic acid, and combinations thereof.

Embodiment 29. The ophthalmic composition according to any of the preceding embodiments, that comprises trehalose in an amount by weight not less than or equal to an amount by weight of D-panthenol.

Embodiment 30. The ophthalmic composition according to any of the preceding embodiments, that comprises trehalose in an amount by weight not less than an amount by weight of D-panthenol.

Embodiment 31. The ophthalmic composition according to any of the preceding embodiments, further comprising an osmolarity regulating agent.

Embodiment 32. The ophthalmic composition according to the preceding embodiment, wherein the osmolarity regulating agent is sodium chloride.

Embodiment 33. The ophthalmic composition according to any of the preceding embodiments, further comprising a buffer.

Embodiment 34. The ophthalmic composition according to the preceding embodiment, wherein the buffer is selected from the group consisting of borate buffer, citrate buffer, phosphate buffer, tris buffer, trometamol/maleic acid.

Embodiment 35. The ophthalmic composition according to any of the preceding embodiments, wherein the composition has pH from 6 to 7.

Embodiment 36. The ophthalmic composition according to the preceding embodiment, whose pH is 6.3.

Embodiment 37. The ophthalmic composition according to any of the embodiments 33-36, that comprises citrate buffer.

Embodiment 38. The ophthalmic composition according to any of the preceding embodiments, that comprises pH regulating agent which is selected from HCl and/or NaOH.

Embodiment 39. The ophthalmic composition according to embodiment 39, which further comprises N-acetylcarnosine.

Embodiment 40. The ophthalmic composition according to any of the preceding embodiments, which is devoid of preservatives and/or of any further pharmaceutically active ingredients.

Embodiment 41. The ophthalmic composition according to any of the preceding embodiments, that is devoid of any further excipients or carriers.

Embodiment 42. The ophthalmic composition according to any of the preceding embodiments, that has dynamic viscosity measured by Brookfield method at 20°C from 1 to 50 mPa•s.

Embodiment 43. The ophthalmic composition according to the preceding embodiment, that has dynamic viscosity measured by Brookfield method at 20°C from 1 to 20 mPa•s.

Embodiment 44. The ophthalmic composition according to the preceding embodiment, that has dynamic viscosity measured by Brookfield method at 20°C from 1.5 to 10 mPa•s.

Embodiment 45. The ophthalmic composition according to any of the preceding embodiments, that is configured in the form of eye drops.

Embodiment 46. A composition as defined in any of the preceding embodiments for use in the prevention and/or treatment of an ophthalmological pathology.

Embodiment 47. The composition for use according to the preceding embodiment, wherein the ophthalmological pathology is selected from the group consisting of dry eye syndrome, Sjögren syndrome, inflammation of the conjunctiva, irritation, itchiness, burning and allergic reactions of the eye, and related conditions.

Embodiment 48. The composition for use according to the preceding embodiment, wherein the ophthalmological pathology is dry eye syndrome.

Throughout the description and claims the phrase "ophthalmic composition" and variations of the phrase are to be understood as referring to eye preparations which are sterile liquid, semi-solid or solid preparations intended for administration upon the eyeball and/or to the conjunctiva, or for insertion in the conjunctival sac as defined in the monography of European Pharmacopoeia 10.0. Furthermore, the phrase "ophthalmic composition" may be defined as an eye preparation which is instilled in the eye.

Throughout the description and claims the word "comprise" and variations of the word are not intended to exclude other technical features, additives, components, or steps. Furthermore, the word "comprise" encompasses the case of "consisting essentially of" and/or "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred aspects and embodiments described herein.

**Examples**

Materials

Experiments were run with dexpanthenol, trehalose dihydrate, sodium hyaluronate with intrinsic viscosity 1.00m$^3$/kg and mean molecular weight 485 kDa.

Formulations

In total, aqueous compositions with different concentration of sodium hyaluronate, dexpanthenol and trehalose were prepared. Some compositions also contained N-acetylcarnosine and/or NaCl and/or citrate buffer and were adjusted to pH 6.3 with HCl or NaOH solution as required. As non-limiting examples, the preparations of said compositions are described in details below.

A composition containing 0.1% or 0.2% or 0.3% or 0.4% or 0.5% sodium hyaluronate, 2% dexpanthenol and 3% trehalose was prepared as follows: firstly, in the beaker 600 mg of sodium citrate dihydrate is dissolved in approx. 200 mL purified water. Then 9000 mg of trehalose dihydrate calculated as trehalose anhydrous is added and stirred as long as clear solution is obtained. Then 300 mg or 600 mg or 900 mg or 1200 mg or 1500 mg of sodium hyaluronate is added slowly and agitated at elevated temperature (50°C) until clear solution is obtained. In the second beaker 6000 mg of dexpanthenol is added to approx. 30 mL of purified water and stirred until clear solution. In the next step the solution with dexpanthenol is added to the first beaker. The mixture is stirred and thereafter solution is filled with purified water up to 300 mL, and the pH is adjusted to 6.3 by using NaOH or HCl solution as required.

A composition containing 0.1% or 0.2% or 0.3% or 0.4% or 0.5% sodium hyaluronate, 2% dexpanthenol and 3% trehalose was prepared as follows: firstly, in the beaker 600 mg of sodium citrate dihydrate is dissolved in approx. 200 mL purified water. Then 9000 mg of trehalose dihydrate calculated as trehalose anhydrous is added and stirred as long as clear solution is obtained. In the next step 3000 mg of N-acetylcarnosine is added and stirred as long as clear solution is obtained. Then 300 mg or 600 mg or 900 mg or 1200 mg or 1500 mg of sodium hyaluronate is added slowly and agitated at elevated temperature (50°C) until clear solution is obtained. In the second beaker 6000 mg of dexpanthenol is added to approx. 30 mL of purified water and stirred until clear solution. In the next step the solution with dexpanthenol is added to the first beaker. The mixture is stirred and thereafter solution is filled with purified water up to 300 mL, and the pH is adjusted to 6.3 by using NaOH or HCl solution as required.

A composition containing 0.1% or 0.2% or 0.3% or 0.4% or 0.5% sodium hyaluronate, 2% dexpanthenol and 3% trehalose was prepared as follows: firstly, in the beaker 180 mg of sodium chloride is dissolved in approx. 200 mL purified water. Then 900 mg of trehalose dihydrate calculated as trehalose anhydrous is added and stirred as long as clear solution is obtained. In the next step 3000 mg N-acetylcarnosine is added and stirred as long as clear solution is obtained. Then 300 mg or 600 mg or 900 mg or 1200 mg or 1500 mg of sodium hyaluronate is added slowly and agitated at elevated temperature (50°C) until clear solution is obtained. In the second beaker 6000 mg of dexpanthenol is added to approx. 30 mL of purified water and stirred until clear solution. In the next step the solution with dexpanthenol is added to the first beaker. The mixture is stirred and thereafter solution is filled with purified water up to 300 mL, and the pH is adjusted to 6.3 by using NaOH or HCl solution as required.

Water binding capacity

The water binding capacity (WBK) was determined as the maximum amount of water imbibed by the sample and retained under low-speed centrifugation (centrifuge Heraeus Sepatech model Labofuge Ae). This effect was measured by the gravimetric method employing a microbalance with d=0.001 mg (balance Sartorius model MSA66P) as describe below.

Sodium hyaluronate, dexpanthenol and trehalose dihydrate were weighted into preweighed Eppendorf vessels at the different proportions as shown in Table 1. Total weight of each sample was 150 mg. The initial total weight of the sample was noted ($w_0$). Subsequently, distilled water in small drops was added, stirred with a needle after each addition until mixture was thoroughly wetted without the effect of flowing. Then the sample was centrifuged at 2000 rpm for 1 minute at ambient temperature. Next, water was added to the sample without mixing until a small water excess was present in the upper part of the sample. Then the sample was centrifuged at 4000 rpm for 5 minutes at ambient temperature. After centrifugation, the slight amount of a supernatant was discarded. Next, the total weight of the sample was determined after removing the supernatant ($w_1$).

The measured water binding capacity (WBK) was determined with the following formula:

$$\text{WBK (gravimetric)} = \frac{(w_1 - w_0) \cdot 100\ \%}{w_0}$$

where:

"$w_0$" = weight of initially total mass of the sample;
"$w_1$" = weight of total mass of the sample after centrifugation and without supernatant.

Next, the theoretical WBK of the combinations was calculated as the sum of the WBK% of pure ingredients in the proportions the ingredients are present in the mixture. This theoretical WBK is the water binding capacity which would have been expected in the case of a purely additive effect.

The deviation of the measured WBK from the calculated theoretical WBK reflects the synergistic effect, which can be calculated according to following equation:

$$Synergy = \frac{WBK\ measured * 100\%}{WBK\ theoretical} - 100$$

Otherwise expressed, the synergistic effect indicates how much one can decrease the concentration of sodium hyaluronate in the combination to maintain the same level of WBK. The synergistic effect can be seen in Table 1.

Table 1. Water biding capacity (WBK) of sodium hyaluronate, dexpanthenol and trehalose and their mixtures.

| Sodium hyaluronate | Dexpanthenol | Trehalose | WBK measured (%) | WBK theoretical (%) | Synergy (%) |
|---|---|---|---|---|---|
| 100% | 0% | 0% | 701.00 | - | - |
| 0% | 100% | 0% | 7.90 | - | - |
| 0% | 0% | 100% | 11.60 | - | - |
| 2.5% | 27.5% | 70% | 88.40 | 27.82 | 217.79 |
| 2.5% | 48.75% | 48.75% | 67.30 | 27.03 | 148.97 |
| 2.5% | 70% | 27.5% | 54.40 | 26.25 | 107.28 |
| 2.5% | 97.5% | 0% | 40.00 | 25.23 | 58.56 |
| 10% | 20% | 70% | 220.90 | 79.80 | 176.82 |
| 10% | 45% | 45% | 209.70 | 78.88 | 165.86 |
| 10% | 70% | 20% | 169.10 | 77.95 | 116.93 |
| 10% | 90% | 0% | 160.80 | 77.21 | 108.26 |

Table 1 shows the increasing synergic effect on WBK when increasing the concentration of trehalose for 2.5 and 10 % sodium hyaluronate concentrations and different concentrations of dexpanthenol. It was observed that the addition of trehalose increased the amount of bound water compared with the composition which only contained sodium hyaluronate and dexpanthenol. The effect became more significant when an amount by weight of trehalose was not less than or equal to an amount by weight of dexpanthenol and even more significant when an amount by weight of trehalose was not less than an amount by weight of dexpanthenol.

Antioxidative activity

Furthermore, the following compositions as shown in Table 2 and prepared as described above were tested for their antioxidative activities.

Table 2. Compositions tested for antioxidative activity.

| | Composition A | Composition B |
|---|---|---|
| Sodium hyaluronate | 4.00mg | 4.00mg |
| Trehalose dihydrate | 30.00mg (calculated as trehalose anhydrous) | 30.00mg (calculated as trehalose anhydrous) |
| Dexpanthenol | 20.00mg | 20.00mg |
| N-acetyl L-carnosine | 10.00mg | - |

(continued)

|  | Composition A | Composition B |
|---|---|---|
| Sodium citrate dihydrate | 2.00mg | 2.00mg |
| NaOH/HCl | q.s. (adjust pH to 6.3) | q.s. (adjust pH to 6.3) |
| Water, purified | ad 1mL | ad 1mL |

The determination of antioxidant activity of the compositions was based on the free radical scavenging method using 2,2-diphenyl-1-picryl-hydrazyl-hydrate (DPPH). The method consists in spectrophotometric measurement by UV-Vis spectrophotometer (Cary 100 UV-Visible Spectrophotometer from Agilent Technologies) concentration of DPPH radical. 5.0 mL of the indicated composition was mixed with 1.0 mL of DPPH solution in methanol (0.04 mg/mL). The mixture was allowed to stand in darkness for 30 minutes. The absorbance of obtained solution was measured at 517 nm after 30 minutes from the preparation of the mixture and one hour from the preparation of the mixture. The output value of the test was absorbance of mixture of 1.0 mL of DPPH solution in methanol (0.04mg/mL) and 5 mL purified water which was measured directly after preparation of the mixture and the result was 0.1661 ($A_0$). Scavenging effect was calculated with the following formula:

$$\text{Scavenging effect [\%]} = [(A_0 - A_1)/A_0] \times 100\%$$

where:

"$A_0$" - the absorbance of mixture of 1.0 mL of DPPH solution in methanol (0.04mg/mL) and 5 mL of purified water measured at time 0

"$A_1$" - the absorbance of mixture of 1.0 mL of DPPH solution in methanol (0.04mg/mL) and 5 mL of Composition A or Composition B

Results of the tests on antioxidative activity measured for the indicated compositions and calculated according to the above formula are shown in Table 3.

Table 3. Antioxidative activity

| Time [h] | Absorbance of DPPH+ Composition A | Scavenging effect for DPPH + Composition A [%] | Absorbance of DPPH+ Composition B | Scavenging effect for DPPH + Composition B [%] |
|---|---|---|---|---|
| 0.5 | 0.1259 | 24.20 | 0.1445 | 13.00 |
| 1 | 0.1217 | 26.73 | 0.1380 | 16.92 |

The above measured antioxidative activities of Composition A and Composition B show that addition of N-actyl L-carnosine to a composition according to the invention additionally increases the antioxidative effect and this effect is not hindered by all remining ingredients of a composition according to the invention.

**Documents cited in the application**

European Pharmacopoeia 10.0 Volume I published July 2019.

Wegener, A. R., Meyer, L. M. & Schönfeld, C. L. Effect of viscous agents on corneal density in dry eye disease. J. Ocul. Pharmacol. Ther. 31, 504-508 (2015).

Creech JL, Do LT, Fatt I, Radke CJ. In vivo tear-film thickness determination and implications for tear-film stability. Curr Eye Res 1998; 17: 1058-1066.

J. M. Tiffany, N. Winter & G Bliss (1989) Tear film stability and tear surface tension, Current Eye Research, 8:5, 507-515.

L. Silvani, A. Bedei, G. De Garcia, S. Remiddi; Arabinogalactan and hyaluronic acid in ophthalmic solution: Experimental effect on xanthine oxidoreductase complex as key player in ocular inflammation (in vitro study); Experimental Eye Research 196 (2020) 108058.

**Claims**

1. An ophthalmic composition comprising:

   - hyaluronic acid or an ophthalmologically acceptable salt thereof in amount of less than or equal to 2% by weight and not less than or equal to 0.01% by weight,
   - an oligosaccharide in amount of less than or equal to 6% by weight and not less than or equal to 0.1% by weight,
   - a compound selected from the group consisting of panthenol, pantothenic acid, an ophthalmologically acceptable salt of pantothenic acid, and combinations thereof in amount of less than or equal to 5% by weight and not less than or equal to 0.1% by weight,
   and an amount of water up to 100% by weight of the composition, wherein the composition further comprises carnosine or its derivative, and combinations thereof,
   and wherein carnosine or its derivative is selected from carnosine, carcinine, anserine, balenine or N-acetylcarnosine.

2. The ophthalmic composition according to claim 1, wherein the composition comprises:

   - hyaluronic acid or an ophthalmologically acceptable salt thereof in amount of less than or equal to 0.6% by weight and/or not less than or equal to 0.1% by weight,
   - an oligosaccharide in amount of less than or equal to 5% by weight and/or not less than or equal to 0.5% by weight,
   - a compound selected from the group consisting of panthenol, pantothenic acid, an ophthalmologically acceptable salt of pantothenic acid, and combinations thereof in amount of less than or equal to 4% by weight and/or not less than or equal to 0.5% by weight, and an amount of water up to 100% by weight of the composition.

3. The ophthalmic composition according to any of claims 1-2, wherein the composition comprises an oligosaccharide in an amount by weight not less than or equal to an amount by weight of a compound selected from the group consisting of panthenol, pantothenic acid, an ophthalmologically acceptable salt of pantothenic acid, and combinations thereof.

4. The ophthalmic composition according to any of claims 1-3, wherein the weight average molecular weight (Mw) of the hyaluronic acid or of the ophthalmologically acceptable salt thereof is from 5 to 2500 kDa.

5. The ophthalmic composition according to any of claims 1-4, wherein the oligosaccharide is a disaccharide.

6. The ophthalmic composition according to claim 5, wherein the disaccharide is selected from the group consisting of cellobiose, gentiobiose, inulobiose, isomaltose, isomaltulose, kojibiose, lactulose, lactose, laminaribiose, leucrose, maltose, maltulose, 2 alpha-mannobiose, 3 alpha-mannobiose, melibiose, nigerose, rutinose, sophorose, sucrose, trehalose, trehalulose, turanose, and any combination thereof.

7. The ophthalmic composition according to any of claims 5-6, wherein the disaccharide is trehalose.

8. The ophthalmic composition according to any of the preceding claims, wherein the composition comprises sodium hyaluronate, trehalose and D-panthenol.

9. The ophthalmic composition according to any of the preceding claims, wherein the weight ratio of hyaluronic acid or an ophthalmologically acceptable salt thereof, to the oligosaccharide, to the compound selected from panthenol, pantothenic acid, an ophthalmologically acceptable salt of pantothenic acid, and combinations thereof, is from 1:1:1 to 1:50:40.

10. The ophthalmic composition according to any of the preceding claims, wherein the composition is devoid of preservatives and/or of any further pharmaceutically active ingredient.

11. The ophthalmic composition according to any of the preceding claims, wherein the composition has pH from 5 to 7.5.

12. The ophthalmic composition according to any of the claims 1-11, wherein the composition is configured in the form of eye drops.

13. A composition as defined in any of the preceding claims for use in the prevention and/or treatment of an ophthalmological pathology selected from the group consisting of dry eye syndrome, Sjögren syndrome, inflammation of the conjunctiva, irritation, itchiness, burning and allergic reactions of the eye, and related conditions.

14. The composition for use according to claim 13, wherein the ophthalmological pathology is dry eye syndrome.

**Patentansprüche**

1. Ophthalmische Zusammensetzung, umfassend:

   - Hyaluronsäure oder ein pharmazeutisch verträgliches Salz davon in einer Menge von weniger als oder gleich 2 Gewichts-% und nicht weniger als oder gleich 0,01 Gewichts-%,
   - ein Oligosaccharid in einer Menge von weniger als oder gleich 6 Gewichts-% und nicht weniger als oder gleich 0,1 Gewichts-%,
   - eine Verbindung, ausgewählt aus der Gruppe bestehend aus Panthenol, Pantothensäure, einem ophthalmologisch verträglichen Salz von Pantothensäure und Kombinationen davon in einer Menge von weniger als oder gleich 5 Gewichts-% und nicht weniger als oder gleich 0,1 Gewichts-%,
   und eine Menge Wasser von bis zu 100 Gewichts-% der Zusammensetzung, wobei die Zusammensetzung ferner Carnosin oder dessen Derivat und Kombinationen davon umfasst,
   und wobei das Carnosin oder dessen Derivat aus Carnosin, Carcinin, Anserin, Balenin oder N-Acetylcarnosin ausgewählt ist.

2. Ophthalmische Zusammensetzung nach Anspruch 1, wobei die Zusammensetzung Folgendes umfasst:

   - Hyaluronsäure oder ein pharmazeutisch verträgliches Salz davon in einer Menge von weniger als oder gleich 0,6 Gewichts-% und/oder nicht weniger als oder gleich 0,1 Gewichts-%,
   - ein Oligosaccharid in einer Menge von weniger als oder gleich 5 Gewichts-% und/oder nicht weniger als oder gleich 0,5 Gewichts-%,
   - eine Verbindung, ausgewählt aus der Gruppe bestehend aus Panthenol, Pantothensäure, einem ophthalmologisch verträglichen Salz von Pantothensäure und Kombinationen davon in einer Menge von weniger als oder gleich 4 Gewichts-% und/oder nicht weniger als oder gleich 0,5 Gewichts-%,

   und eine Menge Wasser von bis zu 100 Gewichts-% der Zusammensetzung.

3. Ophthalmische Zusammensetzung nach einem der Ansprüche 1-2, wobei die Zusammensetzung ein Oligosaccharid in einer Menge in Gewichts-% von nicht weniger als oder gleich einer Menge in Gewichts-% einer Verbindung, die aus der Gruppe bestehend aus Panthenol, Pantothensäure, einem ophthalmologisch verträglichen Salz von Pantothensäure und Kombinationen davon ausgewählt ist, umfasst.

4. Ophthalmische Zusammensetzung nach einem der Ansprüche 1-3, wobei das gewichtsmittlere Molekulargewicht (Mw) der Hyaluronsäure oder des ophthalmologisch verträglichen Salzes davon zwischen 5 bis 2500 kDa beträgt.

5. Ophthalmische Zusammensetzung nach einem der Ansprüche 1-4, wobei das Oligosaccharid ein Disaccharid ist.

6. Ophthalmische Zusammensetzung nach Anspruch 5, wobei das Disaccharid ausgewählt ist aus der Gruppe bestehend aus Cellobiose, Gentiobiose, Inulobiose, Isomaltose, Isomaltulose, Kojibiose, Lactulose, Lactose, Laminaribiose, Leucrose, Maltose, Maltulose, $2\alpha$-Mannobiose, $3\alpha$-Mannobiose, Melibiose, Nigerose, Rutinose, Sophorose, Sucrose, Trehalose, Trehalulose, Turanose und einer beliebigen Kombination davon.

7. Ophthalmische Zusammensetzung nach einem der Ansprüche 5-6, wobei das Disaccharid Trehalose ist.

8. Ophthalmische Zusammensetzung nach einem der vorhergehenden Ansprüche, wobei die Zusammensetzung Natrium-Hyaluronat, Trehalose und D-Panthenol umfasst.

**9.** Ophthalmische Zusammensetzung nach einem der vorhergehenden Ansprüche, wobei das Gewichtsverhältnis von Hyaluronsäure oder einem ophthalmologisch verträglichen Salz davon zu dem Oligosaccharid, zu der Verbindung, die aus der Gruppe bestehend aus Panthenol, Pantothensäure, einem ophthalmologisch verträglichen Salz von Pantothensäure und Kombinationen davon ausgewählt ist, zwischen 1:1:1 und 1:50:40 liegt.

**10.** Ophthalmische Zusammensetzung nach einem der vorhergehenden Ansprüche, wobei die Zusammensetzung frei von Konservierungsmitteln und/oder beliebigen weiteren pharmazeutischen Wirkstoffen ist.

**11.** Ophthalmische Zusammensetzung nach einem der vorhergehenden Ansprüche, wobei die Zusammensetzung einen pH-Wert zwischen 5 und 7,5 aufweist.

**12.** Ophthalmische Zusammensetzung nach einem der Ansprüche 1-11, wobei die Zusammensetzung in der Form von Augentropfen konfiguriert ist.

**13.** Zusammensetzung, wie in einem beliebigen der vorhergehenden Ansprüche definiert, zur Verwendung in der Prävention und/oder Behandlung einer ophthalmologischen Pathologie, die ausgewählt ist aus der Gruppe bestehend aus Trockenes-Auge-Syndrom, Sjögren-Syndrom, Entzündung der Bindehaut, Reizung, Juckreiz, Brennen und allergischen Reaktionen des Auges und verwandten Erkrankungen.

**14.** Zusammensetzung zur Verwendung nach Anspruch 13, wobei die ophthalmologische Pathologie Trockenes-Auge-Syndrom ist.

**Revendications**

**1.** Composition ophtalmique comprenant :

- l'acide hyaluronique ou un sel ophtalmologiquement acceptable de celui-ci en quantité inférieure ou égale à 2 % en poids et non inférieure ou égale à 0,01 % en poids,
- un oligosaccharide en quantité inférieure ou égale à 6 % en poids et non inférieure ou égale à 0,1 % en poids,
- un composé sélectionné dans le groupe constitué par le panthénol, l'acide pantothénique, un sel ophtalmologiquement acceptable de l'acide pantothénique, et des combinaisons de ceux-ci en quantité inférieure ou égale à 5 % en poids et non inférieure ou égale à 0,1 % en poids,
et une quantité d'eau jusqu'à 100 % en poids de la composition, dans laquelle la composition comprend en outre la carnosine ou son dérivé, et des combinaisons de ceux-ci,
et dans laquelle la carnosine ou son dérivé est sélectionné parmi la carnosine, la carcinine, l'ansérine, la balénine ou la N-acétylcarnosine.

**2.** Composition ophtalmique selon la revendication 1, dans laquelle la composition comprend :

- l'acide hyaluronique ou un sel ophtalmologiquement acceptable de celui-ci en quantité inférieure ou égale à 0,6 % en poids et/ou non inférieure ou égale à 0,1 % en poids,
- un oligosaccharide en quantité inférieure ou égale à 5 % en poids et/ou non inférieure ou égale à 0,5 % en poids,
- un composé sélectionné dans le groupe constitué par le panthénol, l'acide pantothénique, un sel ophtalmologiquement acceptable de l'acide pantothénique, et des combinaisons de ceux-ci en quantité inférieure ou égale à 4 % en poids et/ou non inférieure ou égale à 0,5 % en poids,

et une quantité d'eau jusqu'à 100 % en poids de la composition.

**3.** Composition ophtalmique selon l'une quelconque des revendications 1 à 2, dans laquelle la composition comprend un oligosaccharide en une quantité en poids non inférieure ou égale à une quantité en poids d'un composé sélectionné dans le groupe constitué par le panthénol, l'acide pantothénique, un sel ophtalmologiquement acceptable de l'acide pantothénique, et des combinaisons de ceux-ci.

**4.** Composition ophtalmique selon l'une quelconque des revendications 1 à 3, dans laquelle le poids moléculaire moyen en poids (Mw) de l'acide hyaluronique ou du sel ophtalmologiquement acceptable de celui-ci est de 5 à 2500 kDa.

**5.** Composition ophtalmique selon l'une quelconque des revendications 1 à 4, dans laquelle l'oligosaccharide est un disaccharide.

6. Composition ophtalmique selon la revendication 5, dans laquelle le disaccharide est sélectionné dans le groupe constitué par le cellobiose, le gentiobiose, l'inulobiose, l'isomaltose, l'isomaltulose, le kojibiose, le lactulose, le lactose, le laminaribiose, le leucrose, le maltose, le maltulose, le 2 alpha-mannobiose, le 3 alpha-mannobiose, le mélibiose, le nigérose, le rutinose, le sophorose, le saccharose, le tréhalose, le tréhalulose, le turanose, et toute combinaison de ceux-ci.

7. Composition ophtalmique selon l'une quelconque des revendications 5 à 6, dans laquelle le disaccharide est le tréhalose.

8. Composition ophtalmique selon l'une quelconque des revendications précédentes, dans laquelle la composition comprend l'hyaluronate de sodium, le tréhalose et le D-panthénol.

9. Composition ophtalmique selon l'une quelconque des revendications précédentes, dans laquelle le rapport en poids de l'acide hyaluronique ou d'un sel ophtalmologiquement acceptable de celui-ci, à l'oligosaccharide, au composé sélectionné parmi le panthénol, l'acide pantothénique, un sel ophtalmologiquement acceptable de l'acide pantothénique, et des combinaisons de ceux-ci, est de 1 : 1 : 1 à 1 : 50 : 40.

10. Composition ophtalmique selon l'une quelconque des revendications précédentes, dans laquelle la composition est exempte de conservateurs et/ou de tout autre ingrédient pharmaceutiquement actif.

11. Composition ophtalmique selon l'une quelconque des revendications précédentes, dans laquelle la composition a un pH de 5 à 7,5.

12. Composition ophtalmique selon l'une quelconque des revendications 1 à 11, dans laquelle la composition est configurée sous la forme de gouttes ophtalmiques.

13. Composition telle que définie dans l'une quelconque des revendications précédentes pour utilisation dans la prévention et/ou le traitement d'une pathologie ophtalmologique sélectionnée dans le groupe constitué par le syndrome de l'œil sec, le syndrome de Sjögren, l'inflammation de la conjonctive, l'irritation, les démangeaisons, la sensation de brûlure et les réactions allergiques de l'œil, et les affections apparentées.

14. Composition pour utilisation selon la revendication 13, dans laquelle la pathologie ophtalmologique est le syndrome de l'œil sec.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **WEGENER, A. R.** ; **MEYER, L. M.** ; **SCHÖNFELD, C. L.** Effect of viscous agents on corneal density in dry eye disease.. *J. Ocul. Pharmacol. Ther.*, 2015, vol. 31, 504-508
- **L. SILVANI** ; **A. BEDEI** ; **G. DE GARCIA** ; **S. REMIDDI**. Arabinogalactan and hyaluronic acid in ophthalmic solution: Experimental effect on xanthine oxidoreductase complex as key player in ocular inflammation (in vitro study). *Experimental Eye Research*, 2020, vol. 196, 108058
- *CHEMICAL ABSTRACTS*, 9004-61-9

- *CHEMICAL ABSTRACTS*, 16485-10-2
- *CHEMICAL ABSTRACTS*, 599-54-2
- *CHEMICAL ABSTRACTS*, 81-13-0
- *European Pharmacopoeia*, July 2019, vol. I
- **CREECH JL** ; **DO LT** ; **FATT I** ; **RADKE CJ**. In vivo tear-film thickness determination and implications for tear-film stability.. *Curr Eye Res*, 1998, vol. 17, 1058-1066
- **J. M. TIFFANY** ; **N. WINTER** ; **G BLISS**. Tear film stability and tear surface tension. *Current Eye Research*, 1989, vol. 8 (5), 507-515